# DEMANDE DE BREVET EUROPEEN

(11) **EP 4 717 211 A1**
(43) Date de publication de la demande: **01.04.2026**
(21) Numéro de dépôt: 24202393.5
(22) Date de dépôt: 25.09.2024
(51) Int. Cl.: A61B 17/88, A61F 2/46

(54) **IMPLANT OSSEUX EXPANSIBLE DE CHIRURGIE ORTHOPÉDIQUE VÉTÉRINAIRE, SYSTÈME ORTHOPÉDIQUE ET INSTRUMENT ASSOCIÉ**

(71) Demandeur: IN LIFE VET SA, 1630 Bulle (CH)
(72) Inventeur: Lacaze, Guillaume, 1278 La Rippe (CH)
(74) Mandataire: Gsmart

(57) **Abrégé**

La présente invention concerne un Instrument (A) pour l'implantation d'implants (1) osseux expansibles de chirurgie orthopédique humaine, pour la restauration de volume et/ou géométrie d'un os, par une expansion entre une configuration repliée et une configuration déployée de l'implant (1) s'étendant selon un axe longitudinal (L) entre une extrémité proximale (11) apte à coopérer avec ledit instrument d'implantation (A) pour tenir l'implant et une extrémité distale (12) destinée à être insérée en premier dans l'os, ledit instrument (A) comportant une partie principale préhensible par un praticien, une portion (AA) porte-implant comprenant un tube (A0) de préhension s'étendant selon l'axe longitudinal (L) et possédant, à son extrémité distale, des moyens de retenue complémentaires de moyens de coopération de l'implant, pour tenir l'implant par son extrémité proximale (11), **ledit instrument (A) étant caractérisé en ce qu'il comporte** un instrument (Ac) d'injection de fluide, tel que du ciment osseux, dans ledit implant (1) comprenant au moins une cavité apte à recevoir le fluide, ledit instrument d'injection (Ac) étant disposé en arrière de la portion porte-implant (AA) le long de l'axe longitudinal (L) et comprenant au moins une canule (A0, A1, A3) pour acheminer le fluide jusqu'à l'intérieur de l'implant (1) alors qu'il est encore tenu par ladite portion porte-implant (AA).

## Description

La présente demande se rapporte au domaine de la chirurgie, en particulier la chirurgie orthopédique vétérinaire et notamment du traitement d'une structure osseuse affaissée, par une restauration du volume (ou redressement) de cette structure osseuse. La présente demande concerne en particulier des instruments pour l'installation d'implants osseux expansibles permettant de réparer ou restaurer de structures osseuses lésées, notamment dans le rachis pour le traitement (souvent appelé « réduction ») des fractures de compression, notamment vertébrales (VCF pour l'anglais « Vertébral Compression Fracture »).

Dans ce domaine, le problème de la restauration du volume de structure osseuse qui s'est affaissée est bien connu et la littérature abonde de solutions utilisant des implants expansibles capables de passer d'une configuration repliée à une configuration déployée pour restaurer la hauteur de la structure osseuse, de préférence en combinaison avec une injection de ciment de substitution osseuse, dit ciment (ou ciment osseux). De nombreux ciments sont connus et ils présentent tous l'avantage d'être injectables à l'état liquide ou visqueux pendant une certaine durée, puis de durcir (par polymérisation) à l'intérieur de la structure osseuse pour la stabiliser.

Des problèmes majeurs dans ce domaine concernent l'expansion de l'implant pour restaurer une hauteur au tissu osseux lésé et la fuite de ciment, mais également l'installation des implants dans les os, notamment les vertèbres. En effet, il est nécessaire de disposer d'instruments facilitant l'implantation et réduisant autant que possible la durée et la lourdeur de la tâche pour les praticiens.

De nombreuses solutions sont connues de l'art antérieur, comme notamment les demandes de brevet EP3086729, US11540926, EP3747385, EP2572680, EP3958752, EP2693967, EP2405835, US9579130, EP4216836, WO2023122005, WO2022162418, EP3843668 ou US10945861 mais ces solutions présentent divers problèmes de difficulté de manipulation pour le déploiement, de stabilité et de fiabilité une fois déployés. De plus, ces solutions connues s'accompagnent généralement d'une injection de ciment osseux mais ne fournissent aucun enseignement relatif à la fuite de ciment en dehors de l'implant, alors qu'une telle fuite peut être préjudiciable pour les tissus environnants, voire même l'organisme tout entier si les substances chimiques du ciment envahissent le système sanguin. En effet, le ciment comporte en général une ou plusieurs substances chimiques polymérisables, par exemple comme le poly(méthacrylate de méthyle) (PMMA, pour l'anglais poly-methyl-methacrylate) et éventuellement des additifs. De plus, la température atteinte lors de la polymérisation du ciment n'est pas inoffensive car elle est généralement supérieure à 60°.

Il est connu de l'art antérieur, notamment des document EP1308134, US9510877, US8936627 ou EP2467099, des dispositifs de redressement et stabilisation (ou réduction de fracture d'os), notamment du rachis sous la forme de stent, ou sous forme de ballons ou de sacs gonflables poreux comme dans les documents EP1408888 ou EP1379185, éventuellement équipé de plateaux de support comme dans le document US20060100706. De nombreux documents proposent ce genre de stent, c'est-à-dire d'endoprothèse déformable similaire aux endoprothèses, extenseurs ou tuteurs vasculaires, qui sont généralement sous la forme d'un corps tubulaire maillé, le plus souvent métallique et déformable par l'introduction d'un ballon gonflable pour dilater le corps en écartant les mailles, le ballon étant ensuite retiré pour permettre une injection de ciment, qui durcit et forme ainsi une structure de redressement et de stabilisation. Cependant, ces dispositifs présentent l'inconvénient de nécessiter une implantation en deux voire trois temps avec le gonflage du ballon puis l'injection de ciment, ce qui ralentit et complexifie l'opération mais présente également un risque d'affaissement du dispositif entre le dégonflage du ballon et le remplissage du stent par le ciment, en plus de nécessiter plusieurs instruments différents à utiliser successivement, ce qui implique un risque sanitaire accru. D'autre part, ces solutions présentent l'inconvénient de ne pas répondre au problème majeur des fuites de ciment.

Il est également connu, notamment des documents EP1938765, EP2351539 ou WO200434924, des solutions utilisant des implants à structure maillée, en métal à mémoire de forme, qui est contrainte dans une forme repliée pour l'insertion dans le tissu osseux et capable de s'expandre spontanément, lors du relâchement de la contrainte et/ou sous l'effet de la chaleur. Ces solutions présentent l'inconvénient d'une utilisation d'alliages coûteux et une fabrication complexe pour obtenir une mémoire de forme adéquate qui convienne à l'implantation visée, ce qui implique un surcoût en multipliant le nombre d'implants différents nécessaires pour couvrir les différents cas pathologiques, notamment par l'amplitude de la déformation dont est capable le matériau à mémoire de forme. De plus, la force exercée par le retour du métal à sa forme non contrainte n'est souvent pas suffisante pour redresser correctement la structure osseuse qui s'est affaissée, ou du moins limitative. D'autre part, ces solutions présentent également l'inconvénient de ne pas répondre au problème majeur des fuites de ciment.

Il est également connu de l'art antérieur, notamment des documents EP2405835, US9579130, EP2572680 ou EP1956990 des solutions utilisant des implants expansibles utilisant un mécanisme de levier, à la manière d'un cric de voiture (« jack » en anglais) pour restaurer la structure osseuse à une hauteur déterminée. Ces solutions présentent l'avantage de ne pas risquer d'affaissement contrairement à un sent déployés par un ballon qui est retiré avant l'injection de ciment, mais ils présentent également l'inconvénient d'une implantation en deux voire trois temps et que les dimensions de la surface de support pour exercer la force d'expansion sur le tissu osseux est limitée, en comparaison avec les stents notamment. D'autre part, ils présentent également les inconvénients d'être coûteux, difficiles à déployer, de ne pas être ajustables à la morphologie des structures osseuses et de ne pas répondre non plus au problème majeur des fuites de ciment.

D'autres problèmes récurrents en chirurgie orthopédique concernent l'invasivité (c'est-à-dire le but de faire une incision et des lésions les moins étendues possibles) mais également le ratio de déploiement afin d'obtenir un implant déployé qui comble le plus grand volume possible tout en ayant été introduit par un passage le plus petit possible. D'autre part, ce ratio de déploiement va impacter la répartition des forces pour redresser les vertèbres : si on est trop déformable, la pression d'injection du ciment va plutôt déformer la poche au lieu de restaurer la hauteur.

Un problème complémentaire à celui du déploiement concerne le repliement qui n'est généralement pas possible dans les implants de l'art antérieur. La maitrise du repliement permet la maitrise du déploiement et donc du site d'injection avec une répartition homogène du ciment et de la pression pour remplir l'espace à combler suite à l'affaissement. Homothétie parfaite s'adaptant à la fracture en respectant la forme de l'os à l'intérieur de la fracture.

Dans ce contexte, on comprend qu'il persiste dans le domaine de nombreux problèmes techniques liés aux implants et qui s'accompagnent de problèmes concernant les instruments d'implantation qui sont généralement trop nombreux et complexes pour répondre aux problèmes majeurs de l'invasivité et de la facilité et la durée de l'intervention chirurgicale.

Dans ce contexte, un but de la présente invention est de pallier au moins certains inconvénients de l'art antérieur en proposant un instrument d'implantation chirurgicale d'implants osseux expansibles, pour la restauration de structure osseuses affaissées fiable et simple à manipuler et implanter.

Ce but est atteint par un instrument pour l'implantation d'implants osseux expansibles de chirurgie orthopédique vétérinaire, pour la restauration de volume et/ou géométrie d'un os, par une expansion entre une configuration repliée et une configuration déployée de l'implant s'étendant selon un axe longitudinal entre une extrémité proximale apte à coopérer avec ledit instrument d'implantation pour tenir l'implant et une extrémité distale destinée à être insérée en premier dans l'os, ledit instrument comportant une partie principale préhensible par un praticien, une portion porte-implant comprenant un tube de préhension s'étendant selon l'axe longitudinal et possédant, à son extrémité distale, des moyens de retenue complémentaires de moyens de coopération de l'implant, pour tenir l'implant par son extrémité proximale, ledit instrument étant caractérisé en ce qu'il comporte un instrument d'injection de fluide, tel que du ciment osseux, dans ledit implant comprenant au moins une cavité apte à recevoir le fluide, ledit instrument d'injection étant disposé en arrière de la portion porte-implant le long de l'axe longitudinal et comprenant au moins une canule pour acheminer le fluide jusqu'à l'intérieur de l'implant alors qu'il est encore tenu par ladite portion porte-implant.

Selon une autre particularité, l'instrument comporte également un instrument d'expansion de l'implant apte à coopérer avec des moyens d'expansion mécaniques de l'implant, grâce à une tige d'expansion traversant ladite portion porte-implant et ledit tube de préhension pour actionner lesdits moyens d'expansion mécaniques.

Selon une autre particularité, ladite tige d'expansion traverse ledit instrument d'injection jusqu'à l'extrémité distale du tube de préhension.

Selon une autre particularité, les moyens de retenue de l'instrument et les moyens de coopération de l'implant dont ils sont complémentaires comportent un manchon proximal de l'implant autour duquel se fixe lesdits moyens de retenue et comprennent un plot déplaçable dans une gorge en L pour une retenue de l'implant par un mouvement de translation et de rotation, par rapport à l'axe longitudinal, dudit plot dans ladite gorge.

Selon une autre particularité, ledit instrument d'injection comporte un piston actionnable sur l'instrument pour pousser le fluide stocké dans une chambre à l'intérieur de l'instrument, au travers de ladite canule.

Selon une autre particularité, ladite canule d'injection est formée par le tube de préhension débouchant sur un manchon proximal de l'implant.

Selon une autre particularité, ladite canule d'injection est formée par un tube d'injection apte à être inséré à l'intérieur du tube de préhension et débouchant à l'intérieur d'une ouverture d'un manchon proximal de l'implant retenu par le tube de préhension, directement dans une cavité de l'implant ou sur axe de l'implant comprenant un conduit et au moins une ouverture pour répartir ledit fluide dans l'implant.

Selon une autre particularité, ladite canule d'injection est formée par ladite tige d'expansion qui est creuse et apte à être insérée à l'intérieur du tube de préhension et débouchant à l'intérieur d'une ouverture d'un manchon proximal de l'implant retenu par le tube de préhension, directement dans une cavité de l'implant ou sur axe de l'implant comprenant un conduit et au moins une ouverture pour répartir ledit fluide dans l'implant.

Selon une autre particularité, la portion porte-implant comporte un montage solidaire du tube de préhension directement dans l'instrument principal.

Selon une autre particularité, la portion porte-implant comporte un embout amovible possédant un logement apte à être monté sur une protubérance de l'instrument au travers duquel le fluide est acheminé vers l'embout amovible portant le tube de préhension.

Selon une autre particularité, ledit embout amovible formant le porte-implant est retenu sur l'instrument grâce à un plot coopérant avec une gorge en L pour un verrouillage dans un mouvement de translation et de rotation par rapport à l'axe longitudinal.

Selon une autre particularité, la direction de la rotation pour le verrouillage dudit porte-implant sur l'instrument est opposée à la direction de la rotation pour le verrouillage de l'implant sur le porte-implant.

Selon une autre particularité, le porte-implant comporte un loquet de translation disposé de manière amovible dans ladite gorge pour empêcher la translation dudit plot, jusqu'à une injection de fluide et/ou un actionnement de l'expansion provoquant le raccourcissement dudit implant en longueur.

Selon une autre particularité, le porte-implant comporte un loquet de rotation disposé de manière amovible dans ladite gorge pour empêcher la rotation dudit plot jusqu'à ce qu'une désolidarisation du porte-implant et de l'instrument soit souhaitée.

Un autre but de la présente demande est de pallier au moins une partie des inconvénients de l'art antérieur en proposer un système d'intervention chirurgicale facile à utiliser et permettant une stabilisation efficace des tissus osseux

Ce but est atteint par un système de traitement orthopédique de tissu osseux lésé comprenant un ciment de substitution osseuse et au moins un instrument d'implantation et d'injection de ciment dans l'implant, caractérisé en ce qu'il comporte un implant selon l'un des modes de réalisation décrit dans la présente demande.

Selon une autre particularité, l'instrument d'implantation et d'injection de ciment comporte des moyens de contrôle de la pression et/ou de l'aspiration du ciment pour replier l'implant en configuration repliée si nécessaire.

D'autres particularités et avantages de la présente invention apparaîtront plus clairement à la lecture de la description de divers modes de réalisation ci-après, faite en référence aux dessins annexés, dans lesquels :

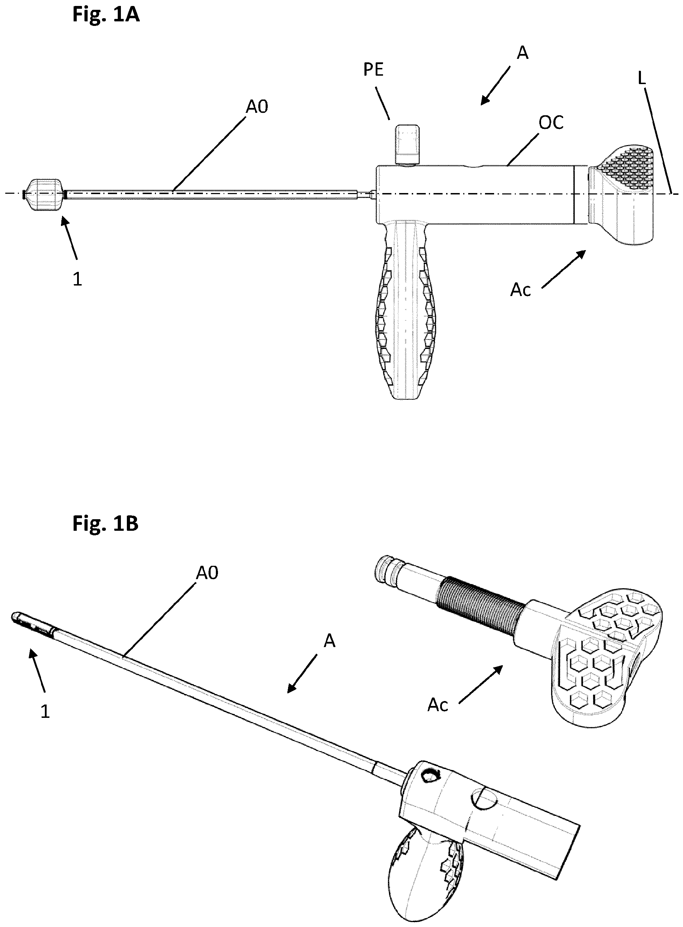

La figure 1A représente une vue de profil d'une instrumentation d'implantation pour un implant expansible orthopédique comprenant un porte un implant un instrument d'injection de ciment et l'implant porté à l'extrémité de l'instrument, et la figure 1B représente une vue en perspective de l'instrumentation de la figure 1A avec l'instrument d'injection de ciment sortie de l'instrument de sortie de porte implant ;

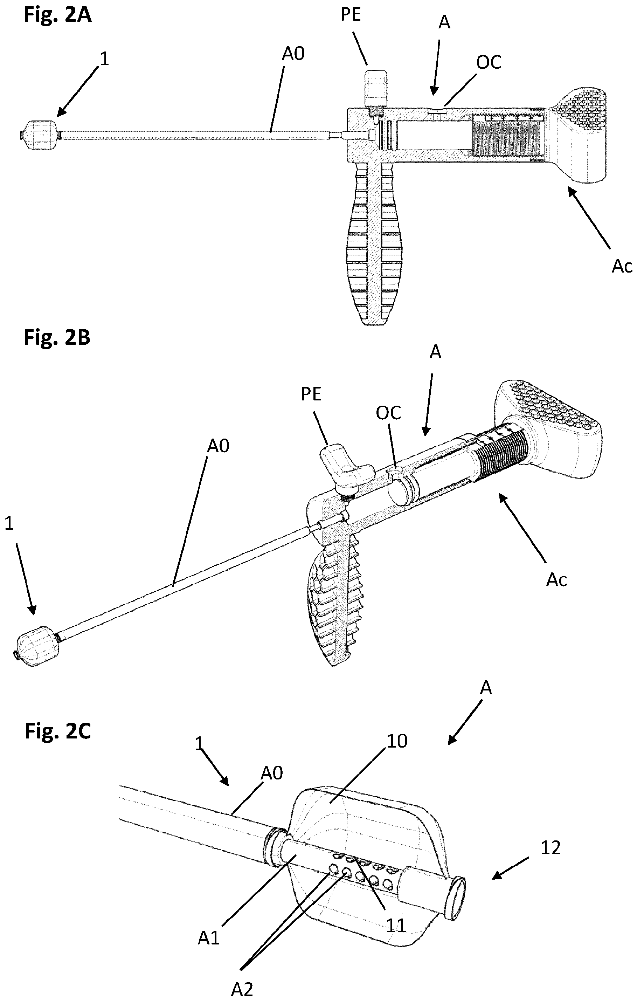

La figure 2A représente une vue en transparence l'instrumentation d'implantation d'implant expansive, la figure 2B représente une vue en perspective avec une partie en couple de l'instrument de la figure 2A et la figure 2C représente une vue en perspective de l'extrémité du porte-implant avec l'implant monté dessus ;

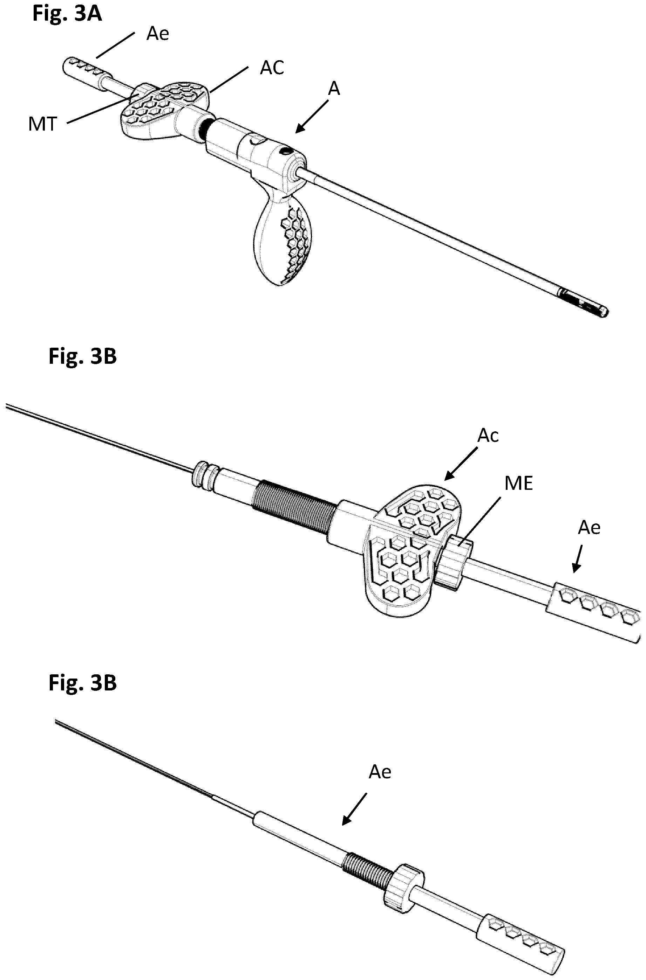

La figure 3A représente une vue en perspective d'un instrument d'expansion d'implant expansible selon certaines modes de réalisations inséré à l'intérieur d'un instrument d'injection de ciment selon certains modes de réalisations, la figure 3B représente l'instrument d'expansion seul ;

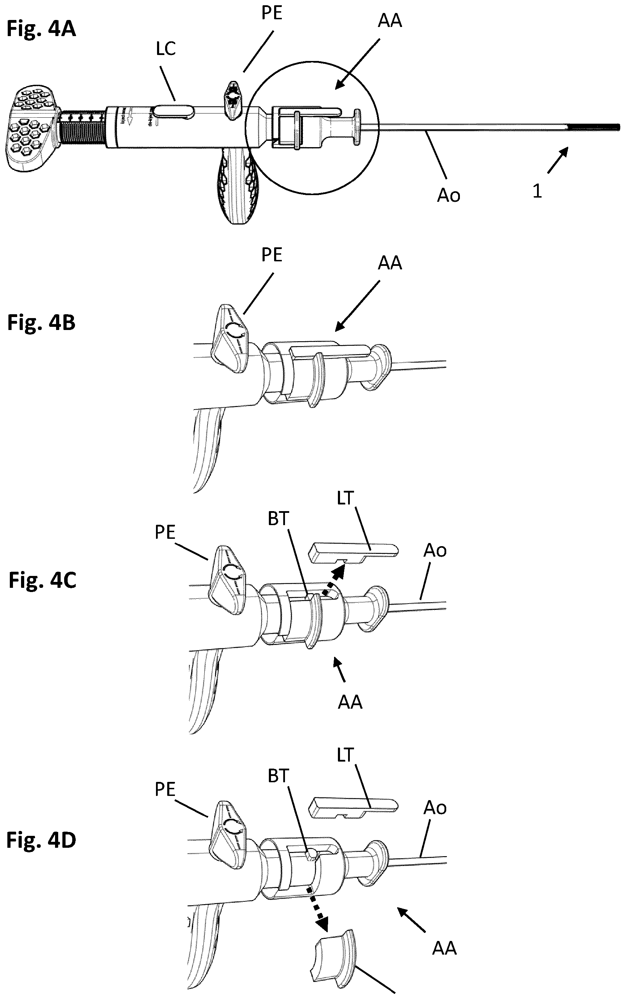

La figure 4A représente une vue en perspective de l'instrumentation d'implantation d'implant expansible comprenant un porte-implant un instrument d'injection de ciment et un instrument de préhension, la figure 4B représente détail de la partie circulaire indiquée sur
la figure 4A et les figures 4C et 4D représentent ce même détail après retrait de verrou sur le porte-implant ;

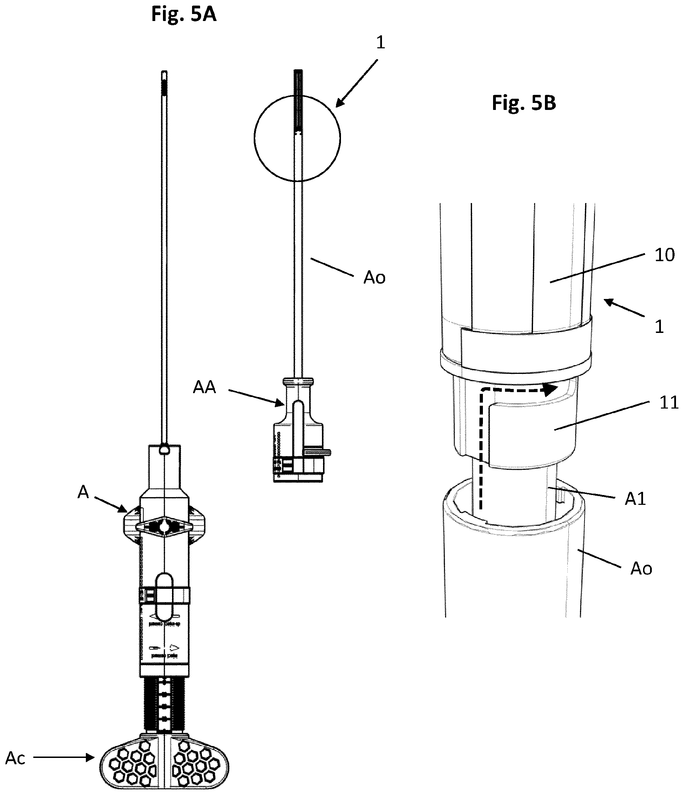

La figure 5A représente une vue de dessus de l'instrumentation d'implantation d'implant d'ancrage osseux et avec le porte-implant désolidarisé de l'instrument principal et la figure 5B représente un détail de la zone circulaire de la figure 5A avec la coopération entre le porte implant et l'implant ;

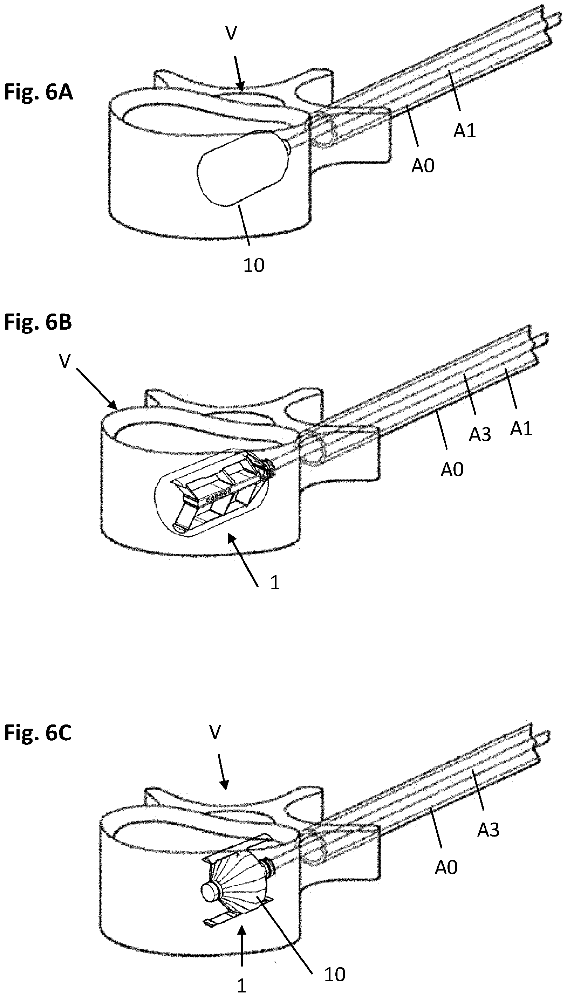

La figure 6A représente une vue en perspective d'une vertèbre dans lequel est inséré un implant osseux expansible selon certains modes de réalisation et les figures 6B et 6C représentent des vues en perspective d'une vertèbre dans laquelle est inséré un implant expansible selon d'autres modes de réalisations ;

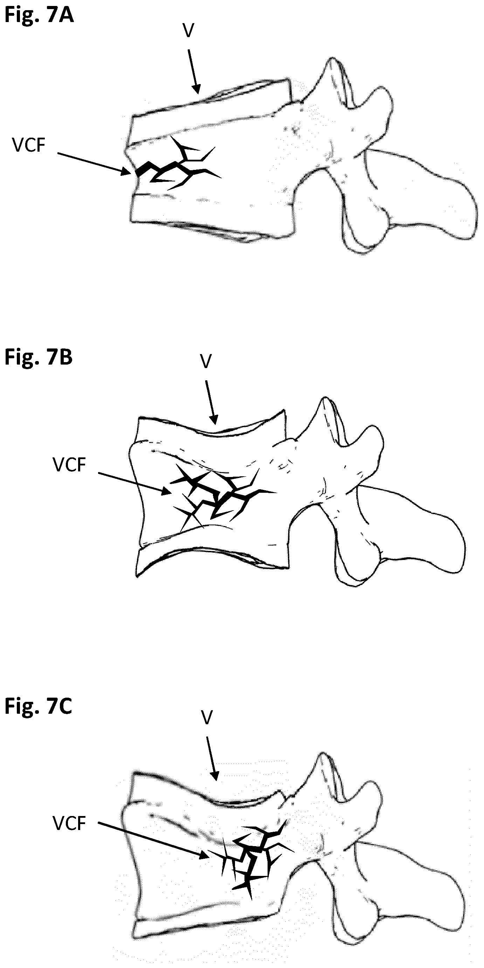

La figure 7A représente une vue de dessus d'une vertèbre dans laquelle est implanté un implant selon divers modes de réalisation, la figure 7B représente une vue de perspective d'une vertèbre dans laquelle est implanté un implant de l'art antérieur et La figure 7C représente une vue en perspective d'une vertèbre dans laquelle est implanté un implant selon certains modes de réalisation ;

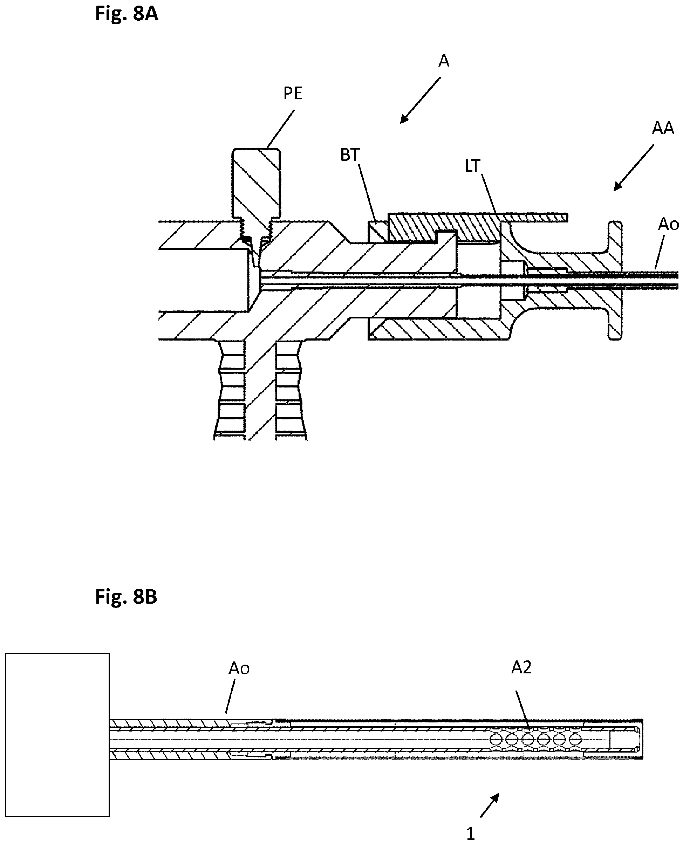

La figure 8A représente une vue en coupe d'une partie d'un instrument d'implantation d'implant osseux expansible du type de celui de la figure 4A, la figure 8B représente une vue en coupe de l'implant porté par un instrument de la figure 8A ;

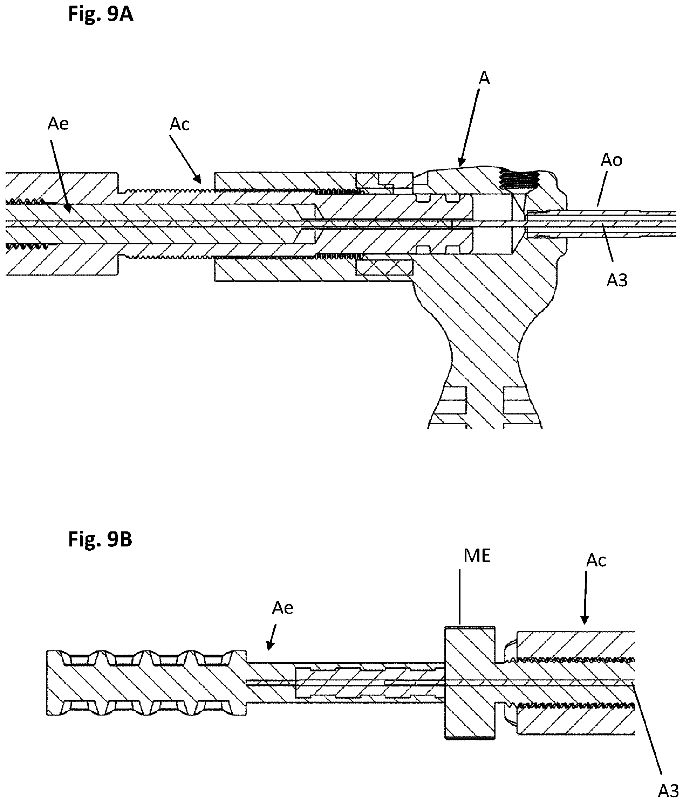

La figure 9A représente une vue en coupe d'une partie d'un instrument d'implantation d'implant expansible du type de celui de la figure 3A et la figure 9B représente une vue en coupe de l'implant tenu par un tel instrument de la figure 9A.

La présente demande concerne un implant et un système de chirurgie orthopédique pour le traitement d'os fracturé et de tissus osseux en général, ainsi qu'un procédé de fabrication de l'implant. L'implant osseux est de préférence un implant Rachidien, et en particulier vertébral ou même en fait intravertébral, mais d'autres utilisations sont envisageables ailleurs dans le rachis (épines intervertébrales) ou dans d'autres structures osseuses où il est nécessaire de combler un espace laissé résultant d'une fracture (dont les causes peuvent être diverses, même si elles impliquent généralement une diminution de densité osseuse). Ainsi, les fractures vertébrales de compression (VCF pour l'anglais Vertébral Compression Fracture) sont une application favorite mais ne sont pas les seules à pouvoir être traitées grâce à la présente invention et l'homme de métier appréciera les possibilités offertes sans nécessiter plus de détails ici. Comme autre os, on peut citer le fémur ou l'humérus (tête) par exemple en cas de risque d'effondrement. La présente demande concerne un implant et un système de chirurgie orthopédique vétérinaire pour le traitement d'os fracturé et de tissus osseux en général, ainsi qu'un procédé de fabrication de l'implant. L'implant osseux est de préférence un implant Rachidien, et en particulier vertébral ou même en fait intravertébral, mais d'autres utilisations sont envisageables ailleurs dans le rachis (épines intervertébrales) ou dans d'autres structures osseuses où il est nécessaire de combler un espace laissé résultant d'une fracture (dont les causes peuvent être diverses, même si elles impliquent généralement une diminution de densité osseuse). Ainsi, les fractures vertébrales de compression (VCF pour l'anglais Vertébral Compression Fracture) sont une application favorite mais ne sont pas les seules à pouvoir être traitées grâce à la présente invention et l'homme de métier appréciera les possibilités offertes sans nécessiter plus de détails ici. Comme autre os, on peut citer le fémur ou l'humérus (tête) par exemple en cas de risque d'effondrement. Dans le domaine vétérinaire, il est connu que les animaux ont des densités osseuses parfois très différentes de l'homme et surtout très variables selon les espèces et même selon les races ou animaux au sein d'une même espèces, notamment pour les chiens dont les propriétés physiques varient énormément d'une race à l'autre. Par exemple, les chiens de type teckel ou similaires ont des vertèbres longues (hautes) mais peu larges comparé aux autres espèces. Il est donc utile de disposer d'implants expansibles qui permettent une forte expansion en hauteur tout en ayant une longueur réduite et il est clairement nécessaire de tenir compte des différences de formes et dimensions des os des différentes espèces pour adapter la thérapie efficacement avec des implants adaptés. De plus, certaines espèces comme le chat ont une corticale osseuse très rigide mais du tissu spongieux plus souple que d'autres espèces. Il est donc nécessaire de prendre également la nature des tissus osseux. Enfin, un autre exemple notable concerne les chevaux qui ont des os, notamment les vertèbres, avec une forme anatomique particulière et qui supportent parfois une charge importante. Selon l'activité (par exemple sportive) et la morphologie, la densité osseuse varie et les implants doivent être adaptés pour permettre leur expansion mais aussi supporter les charges. Ainsi, pour un cheval, il peut être nécessaire d'avoir des implants comprenant plus de bras de support de charge (minimum 3 ou 4) que pour d'autres espèces (où 2 bras support suffisent parfois). En l'absence de bras de support, il est alors nécessaire que l'implant ait une résistance mécanique suffisante, grâce à une capacité à supporter une pression de ciment supérieure à d'autres cas. Par ailleurs, certains modes de réalisation comportant plus de deux plateaux peuvent notamment servir plus efficacement au traitement d'os longs de ce type en répartissant les forces d'expansion sur plus deux surfaces, ce qui fournit une meilleure stabilité quel que soit le type d'os.

Certains modes de réalisation prévoient l'injection d'un fluide (e.g., « ciment osseux », généralement à base d'un polymère comme le PMMA par exemple et bien connu de l'homme de métier, de sorte qu'aucun détail sur le ciment ne sera fourni ici). Ainsi, l'implant une fois positionné peut notamment être stabilisé grâce une telle injection de ciment. Cependant, comme les fuites de ciment restent un problème majeur dans le domaine, divers modes de réalisation proposent de contenir le ciment dans une enveloppe étanche dont le volume après injection peut être maîtrisé grâce à la structure et le matériau de l'enveloppe, en fonction de la pression injectée (et de la configuration des tissus osseux préférentiellement évaluée à l'avance, comme généralement pratiqué dans le domaine). L'étanchéité est bien entendu relative et ce terme n'est pas limitatif non plus, puisque le niveau d'étanchéité est en fait adapté à la viscosité du ciment au moment où on l'injecte. Certains modes de réalisation permettent en particulier un gonflement homothétique de l'enveloppe grâce à la souplesse (relative) de la feuille (10) de matériau métallique biocompatible. Ce matériau est en général un alliage de titane obtenu sous forme de feuille très fine, de préférence par lamination pour un état de surface et une épaisseur maîtrisés, notamment une épaisseur comprise entre 3 et 100 microns, généralement entre 6 et 50 et de préférence 10 et 30 microns. Cette feuille est capable de déformation plastique réversible un nombre de fois satisfaisant pour l'application visée puisqu'elle offre notamment la possibilité de rétracter l'enveloppe formée par la feuille en cas de problème (biocompatibilité et résistance au déchirement). En effet, en général, le pilotage du dosage de ciment permet de surveiller les quinze minutes de polymérisation pendant lesquelles il est possible de rétracter l'enveloppe et aspirer le ciment. D'autre part, par l'injection de ciment, le gonflement de l'enveloppe, l'implant remplit les espaces dans les tissus lésés en fonction des forces de compression et de résistance osseuse par rapport à la pression hydraulique fournie lors de l'injection de ciment. A partir d'une telle feuille, il est nécessaire d'obtenir une structure fermée, ce qui impose déjà de refermer la feuille sur elle-même et de la verrouiller en position. Pour cela, une soudure (ou un collage ou une brasure, ces termes n'étant pas limitatifs ici) peut être réalisée entre deux bords qui se superposent ou sur des bords avec des revers enchevêtrés, pour faciliter et solidifier la soudure. Certains modes de réalisation prévoient donc une fermeture par soudure depuis l'extérieur, simplifié et plus solide grâce à la superposition de couches au niveau de ces plis complémentaires.

Le terme « solidarisé » signifie ici que deux éléments sont rendus solidaires, soit de manière permanente (ou quasi-permanente) mais également parfois qu'une liaison est réalisée pour l'actionnement d'un élément par un autre. Ainsi, un vissage ou une coopération de forme pour verrouiller provisoirement les éléments entre sont couverts par ce terme non limitatif.

Les termes bague, manchon, ou tube désignent des structures creuses telles que des anneaux, des conduits ou des tuyaux mais de façon non limitative, notamment avec des formes diverses (à l'intérieur comme à l'extérieur) bien que la forme cylindrique soit préférée. Le terme canal est en revanche de préférence utilisé ici pour désigner un passage plutôt que l'élément qui le contient et le terme ouverture désigne ici le fait qu'un élément soit ouvert et apte à être traversé en débouchant dans une autre structure ou un autre élément. Généralement les termes manchon et tubes ou conduits désignent des éléments plus longs que les bagues ou anneaux, mais leur utilisation ici n'est pas limitative non plus. D'autre part, les termes douille ou culot désignent également des structures creuses qui sont ouvertes à une extrémité mais fermées à l'autre extrémité, comme des bouchons, des fermetures, des constrictions ou étranglement et ces termes sont utilisés indifféremment sans limitation quelconque.

On notera que l'instrument d'injection de fluide (Ac) peut être muni de moyens de contrôler la pression et/ou la quantité injectée (un manomètre ou au moins des graduations par exemple) et de savoir quel est le volume résultant, afin de contrôler efficacement l'expansion dans les tissus osseux. Avantageusement, des moyens de contrôle de l'air injecté peuvent être présents pour adapter l'injection de ciment en fonction de l'évacuation de l'air dans les tubes creux ou canules des instruments (l'air s'échappant généralement facilement depuis l'implant vers l'instrument grâce au jeu entre les pièces (tiges et tubes ou canules) de l'instrument).

Enfin, on comprend que l'instrumentation proposée dans la présente demande dans certains modes de réalisation, en utilisant un porte-implant (ou ancillaire) relativement classique pour tenir l'implant et l'introduire dans les tissus osseux, mais également moins classique pour l'expandre dans les tissus osseux, présente également l'avantage de pouvoir réaliser toutes les étapes d'implantation et de stabilisation avec un seul instrument et en une opération continue. En effet, l'ancillaire avec un tube creux d'acheminement du ciment au travers du tube qui retient le ciment, permet de disposer d'un instrument permettant d'effectuer l'opération chirurgicale rapidement et efficacement. Après le perçage, on introduit l'implant et sans retirer l'instrument, on peut gonfler l'enveloppe avec le ciment et ensuite retirer l'outil avant, pendant ou même après la polymérisation du ciment (par exemple à l'aide d'un mécanisme de coupe du ciment dur lors d'une rotation de l'instrument). Le temps d l'opération chirurgicale est bien entendu nettement diminué mais également la stabilité de l'implant qui n'est lâché à aucun moment tant qu'il n'est pas stabilisé par l'injection de ciment remplissant tous les volumes libres autour, contrairement à certaines solutions de l'art antérieur.

D'une manière générale, la présente demande concerne un instrument (A) pour l'implantation d'implants (1) osseux expansibles de chirurgie orthopédique vétérinaire, pour la restauration de volume et/ou géométrie d'un os, par une expansion entre une configuration repliée et une configuration déployée de l'implant (1) s'étendant selon un axe longitudinal (L) entre une extrémité proximale (11) apte à coopérer avec ledit instrument d'implantation (A) pour tenir l'implant et une extrémité distale (12) destinée à être insérée en premier dans l'os, ledit instrument (A) comportant une partie principale préhensible par un praticien, une portion (AA) porte-implant comprenant un tube (A0) de préhension s'étendant selon l'axe longitudinal (L) et possédant, à son extrémité distale, des moyens de retenue complémentaires de moyens de coopération de l'implant, pour tenir l'implant par son extrémité proximale (11), ledit instrument (A) étant caractérisé en ce qu'il comporte un instrument (Ac) d'injection de fluide, tel que du ciment osseux, dans ledit implant (1) comprenant au moins une cavité apte à recevoir le fluide, ledit instrument d'injection (Ac) étant disposé en arrière de la portion porte-implant (AA) le long de l'axe longitudinal (L) et comprenant au moins une canule (A0, A1, A3) pour acheminer le fluide jusqu'à l'intérieur de l'implant (1) alors qu'il est encore tenu par ladite portion porte-implant (AA).

Dans certains modes de réalisation, l'instrument (A) comporte également un instrument (Ae) d'expansion de l'implant (1) apte à coopérer avec des moyens d'expansion mécaniques de l'implant (1), grâce à une tige d'expansion (A3) traversant ladite portion porte-implant (AA) et ledit tube (A0) de préhension pour actionner lesdits moyens d'expansion mécaniques. Dans certains de ces modes de réalisation, ladite tige d'expansion (A3) traverse ledit instrument d'injection (Ac) jusqu'à l'extrémité distale du tube (A0) de préhension.

Dans certains modes de réalisation, les moyens de retenue de l'instrument (A) et les moyens de coopération de l'implant (1) dont ils sont complémentaires comportent un manchon proximal de l'implant autour duquel se fixe lesdits moyens de retenue et comprennent un plot déplaçable dans une gorge en L pour une retenue de l'implant par un mouvement de translation et de rotation, par rapport à l'axe longitudinal, dudit plot dans ladite gorge.

Dans certains modes de réalisation, ledit instrument d'injection (Ac) comporte un piston (Pc) actionnable sur l'instrument (A) pour pousser le fluide stocké dans une chambre à l'intérieur de l'instrument (A), au travers de ladite canule (A0, A1).

Dans certains modes de réalisation, ladite canule (A0, A1) d'injection est formée par le tube (A0) de préhension débouchant sur un manchon proximal de l'implant (1). Dans d'autres modes de réalisation, ladite canule (A0, A1) d'injection est formée par un tube (A1) d'injection apte à être inséré à l'intérieur du tube (A0) de préhension et débouchant à l'intérieur d'une ouverture d'un manchon proximal de l'implant (1) retenu par le tube (A0) de préhension, directement dans une cavité de l'implant (1) ou sur axe (3) de l'implant (1) comprenant un conduit (31) et au moins une ouverture (32) pour répartir ledit fluide dans l'implant. Dans encore d'autres modes de réalisation, ladite canule (A0, A1) d'injection est formée par ladite tige d'expansion (A3) qui est creuse et apte à être insérée à l'intérieur du tube (A0) de préhension et débouchant à l'intérieur d'une ouverture d'un manchon proximal de l'implant (1) retenu par le tube (A0) de préhension, directement dans une cavité de l'implant (1) ou sur axe (3) de l'implant (1) comprenant un conduit (31) et au moins une ouverture (32) pour répartir ledit fluide dans l'implant.

Dans certains modes de réalisation, la portion porte-implant (AA) comporte un montage solidaire du tube (A0) de préhension directement dans l'instrument (A) principal. Dans d'autres modes de réalisation, la portion porte-implant (AA) comporte un embout amovible possédant un logement apte à être monté sur une protubérance de l'instrument (A) au travers duquel le fluide est acheminé vers l'embout amovible portant le tube (A0) de préhension.

Dans certains modes de réalisation, ledit embout amovible formant le porte-implant (AA) est retenu sur l'instrument grâce à un plot coopérant avec une gorge en L pour un verrouillage dans un mouvement de translation et de rotation par rapport à l'axe longitudinal (L). Dans certains de ces modes de réalisation, la direction de la rotation pour le verrouillage dudit porte-implant (AA) sur l'instrument (A) est opposée à la direction de la rotation pour le verrouillage de l'implant (1) sur le porte-implant. Dans certains de ces modes de réalisation, le porte-implant (AA) comporte un loquet de translation disposé de manière amovible dans ladite gorge pour empêcher la translation dudit plot, jusqu'à une injection de fluide et/ou un actionnement de l'expansion provoquant le raccourcissement dudit implant (1) en longueur. D'autre part, dans certains de ces modes de réalisation, le porte-implant (AA) comporte un loquet de rotation disposé de manière amovible dans ladite gorge pour empêcher la rotation dudit plot jusqu'à ce qu'une désolidarisation du porte-implant (AA) et de l'instrument (A) soit souhaitée.

Dans certains modes de réalisation, des moyens de verrouillage sont prévus dans certains cas pour empêcher l'implant de se replier. Les diamètres très fins des implants et leurs axes centraux sont difficilement compatibles avec des filetages pour opérer l'expansion par vissage au niveau de l'implant, alors qu'il est avantageux d'effectuer un vissage au niveau de l'instrument actionnant l'expansion, notamment lorsque l'expansion implique un rapprochement des bras support les uns par rapport aux autres. Ainsi, comme connu de l'art antérieur, il est possible d'utiliser par exemple une bague fendue logée dans un renforcement circulaire de l'implant et coopérant avec des crans sur l'axe de poussée ou de traction qui sont orientés pour permettre le passage de cet axe dans un seul sens. Ainsi, l'actionnement de l'axe pour l'expansion des plateaux peut être réalisé par passage successifs des crans, ce qui permet de verrouiller l'implant en configuration déployée.

Cependant, contrairement à certains implants de l'art antérieur où l'axe de traction permettant de rapprocher doit nécessairement rester en place, les implants de la présente demande sont déployés sans rapprochement des bras de support, ce qui permet avantageusement de pouvoir les verrouiller avec un verrou vissé et de ne pas nécessiter de tels crans rendant la tâche difficile et offrant une fiabilité réduite. Ainsi, il est possible d'utiliser par exemple un manchon fileté configuré pour être disposé dans le tube creux de l'instrument d'implantation (A) tenant l'implant (et entourer l'éventuel conduit d'injection de fluide présent à l'intérieur). Un tel manchon possède alors un filetage prévu pour coopérer avec un taraudage de l'extrémité proximale de l'implant et présente des moyens d'actionnement pour le vissage ou dévissage.

Dans certains modes de réalisation, l'instrument d'implantation (A) et d'injection de ciment (Ac) comporte des moyens de contrôle de la pression et/ou de l'aspiration du ciment pour replier l'implant en configuration repliée si nécessaire.

Dans certains modes de réalisation, l'instrument d'implantation (A) est distinct mais complémentaire de l'instrument d'injection (Ac) dont le canal d'injection du ciment traverse un canal à l'intérieur de la tige de l'instrument d'implantation (A) tenant l'extrémité proximale de l'implant (1) grâce à son extrémité distale.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un autre mode de réalisation à moins que l'inverse ne soit explicitement mentionné ou qu'il ne soit évident que ces caractéristiques sont incompatibles ou que la combinaison ne fournisse pas une solution à au moins un des problèmes techniques mentionnés dans la présente demande. De plus, les caractéristiques techniques décrites dans un mode de réalisation donné peuvent être isolées des autres caractéristiques de ce mode à moins que l'inverse ne soit explicitement mentionné.

### Liste détaillée des références dans les figures :

- 1: implant
- 10: feuille
- 11: extrémité proximale
- 101: pli antiforme
- 102: pli synforme
- 110: soudure proximale
- 12: extrémité distale
- 120: soudure distale (liaison étanche)
- 121: fixation par compression (e.g., bague fendue)
- 3: axe central
- 31: conduit dans l'axe central
- 32: ouvertures du conduit de l'axe central
- 13: premier plateau
- 14: deuxième plateau
- 15: troisième plateau
- 20: bague d'expansion
- 131: bras support du premier plateau
- 141: bras support du deuxième plateau
- 151: bras support du troisième plateau
- 132: bras d'expansion du premier plateau
- 142: bras d'expansion du deuxième plateau
- 152: bras d'expansion du troisième plateau
- 130: bras support central du premier plateau
- 140: bras support central du deuxième plateau
- 150: bras support central du troisième plateau
- A: instrument d'implantation
- Ac: instrument d'injection de fluide
- A1: tube creux de préhension
- A3: tige d'expansion?
- TG: tige de guidage
- GR: guide de recouvrement
- PP: Platine de pré-pliage
- ET: tige étoilée
- CP: came de pré-pliage
- CP1: premier angle de came de pré-pliage
- CP2: deuxième angle de came de pré-pliage
- RC: rampe de came
- VV: verrou vissé
- VC: verrou cranté

## Revendications

1. Instrument (A) pour l'implantation d'implants (1) osseux expansibles de chirurgie orthopédique vétérinaire, pour la restauration de volume et/ou géométrie d'un os, par une expansion entre une configuration repliée et une configuration déployée de l'implant (1) s'étendant selon un axe longitudinal (L) entre une extrémité proximale (11) apte à coopérer avec ledit instrument d'implantation (A) pour tenir l'implant et une extrémité distale (12) destinée à être insérée en premier dans l'os, ledit instrument (A) comportant une partie principale préhensible par un praticien, une portion (AA) porte-implant comprenant un tube (A0) de préhension s'étendant selon l'axe longitudinal (L) et possédant, à son extrémité distale, des moyens de retenue complémentaires de moyens de coopération de l'implant, pour tenir l'implant par son extrémité proximale (11), ledit instrument (A) étant **caractérisé en ce qu'il comporte** un instrument (Ac) d'injection de fluide, tel que du ciment osseux, dans ledit implant (1) comprenant au moins une cavité apte à recevoir le fluide, ledit instrument d'injection (Ac) étant disposé en arrière de la portion porte-implant (AA) le long de l'axe longitudinal (L) et comprenant au moins une canule (A0, A1, A3) pour acheminer le fluide jusqu'à l'intérieur de l'implant (1) alors qu'il est encore tenu par ladite portion porte-implant (AA).

2. Instrument (A) selon la revendication 1, **caractérisé en ce qu'**il comporte également un instrument (Ae) d'expansion de l'implant (1) apte à coopérer avec des moyens d'expansion mécaniques de l'implant (1), grâce à une tige d'expansion (A3) traversant ladite portion porte-implant (AA) et ledit tube (A0) de préhension pour actionner lesdits moyens d'expansion mécaniques.

3. Instrument (A) selon la revendication 2, **caractérisé en ce que** ladite tige d'expansion (A3) traverse ledit instrument d'injection (Ac) jusqu'à l'extrémité distale du tube (A0) de préhension.

4. Instrument (A) selon l'une des revendications précédentes, **caractérisé en ce que** les moyens de retenue de l'instrument (A) et les moyens de coopération de l'implant (1) dont ils sont complémentaires comportent un manchon proximal de l'implant autour duquel se fixe lesdits moyens de retenue et comprennent un plot déplaçable dans une gorge en L pour une retenue de l'implant par un mouvement de translation et de rotation, par rapport à l'axe longitudinal, dudit plot dans ladite gorge.

5. Instrument (A) selon l'une des revendications précédentes, **caractérisé en ce que** ledit instrument d'injection (Ac) comporte un piston (Pc) actionnable sur l'instrument (A) pour pousser le fluide stocké dans une chambre à l'intérieur de l'instrument (A), au travers de ladite canule (A0, A1).

6. Instrument (A) selon l'une des revendications précédentes, **caractérisé en ce que** ladite canule (A0, A1) d'injection est formée par le tube (A0) de préhension débouchant sur un manchon proximal de l'implant (1).

7. Instrument (A) selon l'une des revendications 1 à 5, **caractérisé en ce que** ladite canule (A0, A1) d'injection est formée par un tube (A1) d'injection apte à être inséré à l'intérieur du tube (A0) de préhension et débouchant à l'intérieur d'une ouverture d'un manchon proximal de l'implant (1) retenu par le tube (A0) de préhension, directement dans une cavité de l'implant (1) ou sur axe (3) de l'implant (1) comprenant un conduit (31) et au moins une ouverture (32) pour répartir ledit fluide dans l'implant.

8. Instrument (A) selon l'une des revendications 2 à 5, **caractérisé en ce que** ladite canule (A0, A1) d'injection est formée par ladite tige d'expansion (A3) qui est creuse et apte à être insérée à l'intérieur du tube (A0) de préhension et débouchant à l'intérieur d'une ouverture d'un manchon proximal de l'implant (1) retenu par le tube (A0) de préhension, directement dans une cavité de l'implant (1) ou sur axe (3) de l'implant (1) comprenant un conduit (31) et au moins une ouverture (32) pour répartir ledit fluide dans l'implant.

9. Instrument (A) selon l'une des revendications précédentes, **caractérisé en ce que** la portion porte-implant (AA) comporte un montage solidaire du tube (A0) de préhension directement dans l'instrument (A) principal.

10. Instrument (A) selon l'une des revendications 1 à 8, **caractérisé en ce que** la portion porte-implant (AA) comporte un embout amovible possédant un logement apte à être monté sur une protubérance de l'instrument (A) au travers duquel le fluide est acheminé vers l'embout amovible portant le tube (A0) de préhension.

11. Instrument (A) selon la revendication 10, **caractérisé en ce que** ledit embout amovible formant le porte-implant (AA) est retenu sur l'instrument grâce à un plot coopérant avec une gorge en L pour un verrouillage dans un mouvement de translation et de rotation par rapport à l'axe longitudinal (L).

12. Instrument (A) selon la revendication 11, **caractérisé en ce que** la direction de la rotation pour le verrouillage dudit porte-implant (AA) sur l'instrument (A) est opposée à la direction de la rotation pour le verrouillage de l'implant (1) sur le porte-implant.

13. Instrument (A) selon l'une des revendications 10 et 11, **caractérisé en ce que** le porte-implant (AA) comporte un loquet de translation disposé de manière amovible dans ladite gorge pour empêcher la translation dudit plot, jusqu'à une injection de fluide et/ou un actionnement de l'expansion provoquant le raccourcissement dudit implant (1) en longueur.

14. Instrument (A) selon l'une des revendications 10 et 11, **caractérisé en ce que** le porte-implant (AA) comporte un loquet de rotation disposé de manière amovible dans ladite gorge pour empêcher la rotation dudit plot jusqu'à ce qu'une désolidarisation du porte-implant (AA) et de l'instrument (A) soit souhaitée.
